# EUROPÄISCHE PATENTSCHRIFT

(11) **EP 1 584 305 B1**
(45) Veröffentlichungstag und Bekanntmachung des Hinweises auf die Patenterteilung: **05.05.2010**
(21) Anmeldenummer: 05007792.4
(22) Anmeldetag: 08.04.2005
(51) Int. Cl.: A61F 2/84, A61B 17/00, A61B 17/11, A61M 25/00

(54) **In eine Ader des Blutkreislaufs einführbarer Katheter**
Catheter insertable in a blood vessel
Cathéter insérable dans un vaiseau sanguin

(30) Priorität: 08.04.2004 EP 04008545
(43) Veröffentlichungstag der Anmeldung: 12.10.2005
(73) Patentinhaber: KRAUTH medical KG (GmbH & Co.), 22041 Hamburg (DE); Albert-Ludwigs-Universität Freiburg, Körperschaft des öffentlichen Rechts, ges. vertr. d. Prof.Dr.Dr.h.c. W. Jäger, 79085 Freiburg (DE)
(72) Erfinder: Höhmann, Reinhard, 22359 Hamburg (DE); Madsen, Tommy, 22399 Hamburg (DE); Schlensak, Christian, 79194 Gundelfingen (DE); Sarai, Koppany, 79241 Ihringen (DE); Doenst, Torsten, 79108 Freiburg (DE)
(74) Vertreter: Glawe, Delfs, Moll

(56) Entgegenhaltungen:
- WO-A-01/51114
- DE-A1- 19 807 354
- US-B1- 6 406 491

## Beschreibung

Die Erfindung betrifft einen in eine Ader des Blutkreislaufs einführbaren Katheter, insbesondere zur Myokardrevaskularisation durch partielle Arterialisierung des Koronarsinus, der ein Konduit mit wenigstens einem expandierbaren Verankerungselement an seinem distalen Ende zum Verankern an der Aderwand und eine schlauchförmige Hülse (Sheet) aufweist, die auf das Konduit aufgeschoben ist, nach Einbringung des Konduits in die Ader nach proximal wenigstens teilweise zurückziehbar ist und dabei das wenigstens eine Verankerungselement freigibt, so dass dieses expandieren kann.

Die Folgen der koronaren Herzerkrankung mit Einengung oder Verschluss von Herzkranzgefäßen sind mit ca. 30% aller Todesfälle die häufigste Todesursache in allen Industrieländern. In Deutschland sterben jährlich über 340.000 Menschen an den Folgen der koronaren Herzerkrankung. Die aorto-koronare Bypass-Operation ist ein etabliertes Therapieverfahren zur Behandlung der koronaren Herzerkrankung, insbesondere wenn mehrere Kranzadern von der Erkrankung betroffen sind. In Deutschland werden pro Jahr etwa 70.000 Bypass-Operationen durchgeführt. Rund 60% der Patienten, die sich zum ersten Mal einer Bypass-Operation unterziehen, sind zwischen 50 und 69 Jahre alt.

Die aorto-koronare Bypass-Operation wird in aller Regel nach Eröffnung des Brustkorbes und Anschluss des Patienten an die Herz-Lungenmaschine durchgeführt. Üblicherweise wird das Herz während des Eingriffes nach Abklemmung der Hauptschlagader für ca. 30 bis 50 Minuten stillgestellt.

Trotz erfolgreicher, primärer chirurgischer Behandlung lässt sich die koronare Herzerkrankung in den meisten Fällen nicht aufhalten und kann ebenfalls auf die angelegten Bypass übergehen. Rund 30% der Bypass sind 15 Jahre nach Anlage wieder verschlossen. Eine erneute, zweite Bypass-Operation ist mit einem deutlich erhöhtem Operationsrisiko und einer Sterblichkeitsrate von etwa 10% verbunden. Das hohe Operationsrisiko einer Zweit-Operation ist begründet durch: 1. Eine mögliche Verletzung des Herzmuskels oder der großen Gefäße bei der Freilegung des in Narbengewebe eingebackenen Herzens. 2. Eine mögliche Verletzung von noch funktionstüchtigem Bypass während der Freilegung des Herzens und daraus resultierendem Herzinfarkt.

Bei vorangeschrittener koronarer Herzerkrankung können die Herzkranzgefäße in ihrem Verlauf so massiv verändert sein, dass das Aufnähen eines Bypass auf die Kranzader technisch nicht möglich ist. Für diese Patientengruppe steht zur Zeit kein etabliertes Therapieverfahren zur Verfügung. Die Herzlaser-Behandlung, die 1994 für Patienten in Deutschland eingeführt wurde, denen mit konventionellen Möglichkeiten (Bypass-OP) nicht mehr geholfen werden konnte, wird nur noch in Ausnahmefällen durchgeführt. Ein möglicher Nutzen für den Patienten konnte bisher nicht nachgewiesen werden.

Für Patienten, die mit konventionellen Operationstechniken nicht mehr behandelt werden können oder bei denen das Operationsrisiko einer Bypass-Operation extrem erhöht ist, wurde das Konzept der Myokardrevaskularisation durch partielle Arterialisierung des Koronarsinus entwickelt.

Bei diesem Verfahren wird ohne den Einsatz der Herz-Lungenmaschine über einen kleinen Hautschnitt an der rechten Halsseite ein Kathetersystem in die Halsvene eingebracht und in die rechte Vorkammer des Herzens vorgeschoben. Das Kathetersystem trägt einen patienteneigenen Venenbypass, oder eine schlauchförmige Prothese aus einem bekannten Material, wie z.B. Dacron, PTFE o.a., d. h. einen Konduit, das bis in den Koronarsinus geführt und dort verankert wird. Danach wird das freie Ende des Konduits durch die rechte Vorkammer in die Halsvene zurückgezogen und über diese ausgeleitet. Anschließend wird das freie ausgeleitete Ende des Konduits mit der Halsschlagader, die direkt neben der Halsvene gelegen ist, verbunden. Somit wird ein Blutstrom von einer zentralen Arterie zum Koronarsinus etabliert, d. h. ein Blutstrom, der der Strömungsrichtung des Koronarsinus entgegengesetzt ist.

Der Zugang zum Koronarsinus bereitet dabei keine speziellen Probleme. Dieser Zugang ist z. B. bei der Enzymmessung im Falle eines Infarkts oder bei der Platzierung von Herzschrittmachersonden bekannt.

Damit das Konduit seine Funktion erfüllen kann, ist es erforderlich, dass sein distales Ende in der koronaren Vene zuverlässig verankert wird. Zu diesem Zweck können Katheter der eingangs genannten Art verwendet werden (US 6 406 491 B1). Der Katheter wird in die Ader eingeschoben. Hat er seine richtige Stellung erreicht, wird die Hülse zurückgezogen. Dadurch werden die Verankerungselemente freigegeben, so dass das distale Ende in der Wand verankert wird. Das Problem, das dabei auftritt, ist, dass die Verankerung der Zugkraft widerstehen muss, die durch das Zurückziehen der Hülse ausgeübt wird. Dabei kann die empfindliche Ader verletzt oder sogar das distale Ende des Konduits aus seiner Verankerung in der Ader herausgerissen werden.

Die Aufgabe der Erfindung besteht in der Schaffung eines Katheters, bei dem eine Lösung der Verankerung und Beschädigung der Ader beim Herausziehen der Hülse vermieden werden kann.

Die erfindungsgemäße Lösung besteht darin, dass das Konduit an seinem distalen Ende mit einer Verengung seines Lumens versehen ist und der Katheter ein auf einen Führungsdraht aufschiebbares röhrenförmiges Element (Pusher) aufweist, das an seinem vorderen Ende gegen die Verengung anliegt.

Der erfindungsgemäße Katheter kann wie ein vorbekannter Katheter in die Ader auf einem vorher eingebrachten Führungsdraht eingeschoben werden. Wird dann die Hülse zurückgezogen, um das oder die Verankerungselemente freizugeben, wird das distale Ende des Konduits durch das röhrenförmige Element (Pusher) an seinem Ort festgehalten, so dass keine Zugkräfte auf die Verankerung wirken. Anschließend kann dann das röhrenförmige Element aus dem Konduit herausgezogen werden. Dabei treten keine großen Zugkräfte mehr auf, da das röhrenförmige Elemente auf dem Führungsdraht und innerhalb der Konduits ohne großen Widerstand gleiten kann, was zum Beispiel durch geeignete Oberflächenbeschichtung erreicht werden kann.

Als Verankerungselemente können federnde Elemente, insbesondere elastische Haken verwendet werden, die sich in die Wand der Ader einhaken, wenn die Hülse zurückgezogen wird. Die Haken erstrecken sich dabei in vorteilhafter Weise vom Konduit nach distal und nach außen. In diesem Fall ist eine Repositionierung möglich, indem die Hülse erneut nach vorne geschoben wird und die Haken zusammen drückt, wonach dann der Katheter weiter hineingeschoben oder herausgezogen werden kann. Bei einer anderen vorteilhaften Ausführungsform, bei der sich die Haken vom Konduit nach proximal und nach außen erstrecken, ist dies nicht möglich. Dafür hat man aber größere Sicherheit gegen unbeabsichtigtes Herausziehen, da die Haken in diesem Falle fester in die Wand der Ader eingreifen.

Es sind zwar Katheter bekannt, die einen Pusher, eine verschiebbare Hülse und sich spreizende hakenförmige Elemente aufweisen (US 6 241 738 B1). Dabei handelt es sich aber um Werkzeuge, mit denen in die Ader einzubringende bzw. eingebrachte Objekte z.B. entfernt werden sollen. Die hakenförmigen Elemente sind nicht dazu bestimmt, an der Ader anzugreifen. Die Werkzeuge sind nicht dazu bestimmt, im Körper zu verbleiben.

Wenn die Haken an ihren vorderen Enden abgerundet sind, wird eine Verletzung der Ader vermieden. Bei einer vorteilhaften Ausführungsform sind die Haken nur einseitig am Umfang des Konduits vorgesehen. Sie können so gut im Myokard verankert werden war, während die andere Seite der Vene, die sehr dünn ist, nicht beschädigt wird.

Bei einer alternativen Ausführungsform ist das Verankerungselement eine Spirale, die sich nach Zurückziehen der Hülse expandiert.

Das röhrenförmige Element ist vorteilhafter Weise aus Kunststoff oder kohlenstoffähnlichem Material, insbesondere Polytetrafluorethylen, was den Vorteil besonders geringer Reibungskräfte hat. Eine andere vorteilhafte Ausführungsform zeichnet sich dadurch aus, dass das röhrenförmige Element aus Metall, insbesondere einem sehr flexiblen, dünnwandigen Metallrohr (Kanülenrohr) besteht.

Bei einer vorteilhaften besonders einfachen Ausführungsform ist die Verengung Teil des Konduits. Bei einer anderen vorteilhaften Ausführungsform ist die Verengung in Form eines Kopfstücks ausgebildet, das auf das distale Ende des Konduits aufgesetzt ist.

In beiden Fällen sind in vorteilhafter Weise die Verengung und das distale Ende des röhrenförmigen Elements mit komplementären Drehverbindungseinrichtungen versehen. Durch Drehen des röhrenförmigen Elements kann dieses mit der Verengung verbunden werden, so dass das röhrenförmige Element nicht nur eine Druckkraft, sondern auch eine Zugkraft zum Repositionieren des Katheters ausüben kann. Die Drehverbindungseinrichtungen können dabei Gewinde oder Bajonettverbindungen sein. Die Verbindung des röhrenförmigen Elements mit dem Konduit beziehungsweise einem Kopfstück des Konduits kann auch einseitig (exzentrisch) ausgebildet sein.

Bei einigen Ausführungsformen legt sich das Konduit an seinem distalen Ende dichtend an die Wand der Ader an, so dass im Falle der eingangs genannten Anwendung in der Gegenrichtung kein Blut strömen kann. In anderen Fällen kann es aber vorteilhaft sein, wenn zumindest eine gewisse Rückströmung in der Gegenrichtung möglich ist. In diesem Falle liegt das distale Ende des Konduits bei anderen Ausführungsformen nicht dichtend an der Wand der Ader an.

Die Erfindung wird im Folgenden anhand von vorteilhaften Ausführungsformen unter Bezugnahme auf die beigefügten Zeichnungen beispielsweise beschrieben. Es zeigen:
- Fig. 1a und 1b: eine schematische Darstellung des Herzens mit den relevanten Blutgefäßen;
- Fig. 2: in schematischer Darstellung das Prinzip des Einbringens eines Konduits;
- Fig. 3: im Querschnitt der distale Endbereich eines Katheters, der in eine Ader eingeschoben ist, bevor die Hülse zurückgezogen ist;
- Fig. 4: in ähnlicher Darstellung sie in Fig. 3 den distalen Endbereich eines Katheters, nachdem die Hülse zum Freigeben der Verankerungselemente teilweise zurückgezogen ist;
- Fig. 5: in ähnlicher Darstellung wie in den Fig. 3 und 4 eine alternative Ausführungsform;
- Fig. 6: eine Ausführungsform mit am Ende abgerundeten Verankerungselemente;
- Fig. 7: in ähnlicher Darstellung wie in den Fig. 3 bis 5 eine Ausführungsform mit einem alternativen Verankerungselement;
- Fig. 8: ein Konduit mit einem verengten distalen Endbereich;
- Fig. 9: eine alternative Ausführungsform mit einer Drehverbindungseinrichtung von Pusher und Kopfstück; und
- Fig. 10-12: alternative Ausführungsformen der Verengung beziehungsweise Drehverbindungseinrichtung.

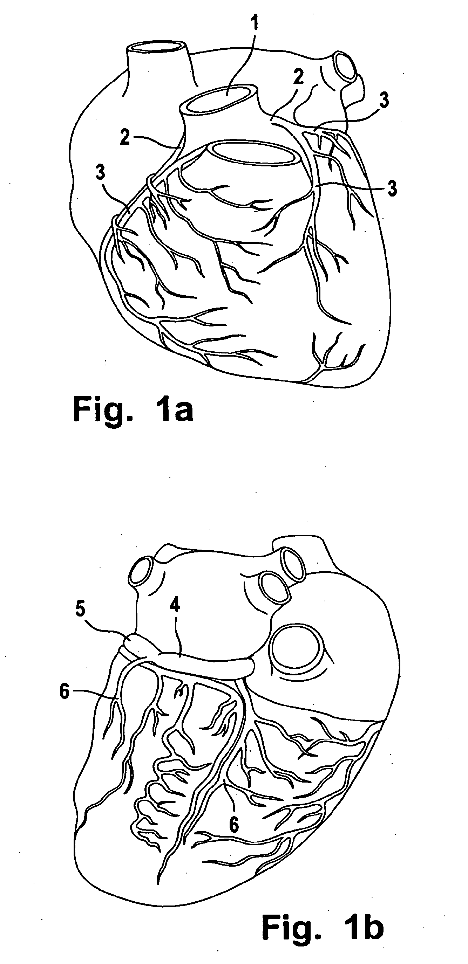

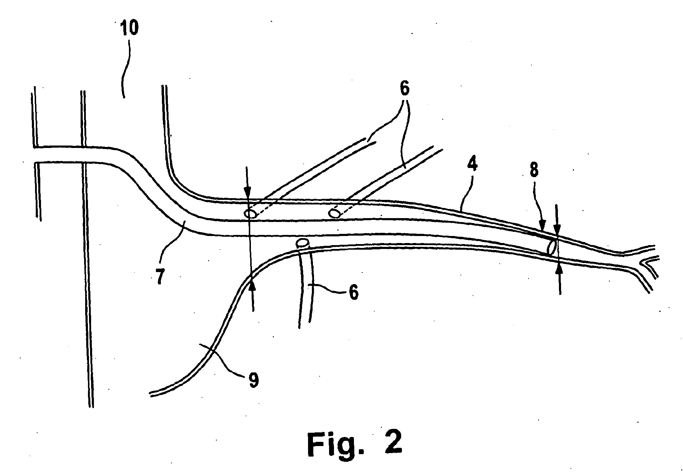

In Fig. 1a und b ist schematisch ein menschliches Herz gezeigt. Aus der Aorta 1 strömt u.a. durch eine Öffnung 2 Blut in die Herzkranzgefäße 3 und versorgt damit die Herzmuskeln. Das zurückfließende Blut kommt aus der Koronarvene 5 und diversen Seitenästen 6, sammelt sich im Koronarsinus 4 und fließt ab in den rechten Vorhof. Bei vorangeschrittener koronarer Herzerkrankung oder bei Rezidivoperationen können die Herzkranzgefäße in Ihrem Verlauf so massiv verändert sein, dass das Aufnähen eines Bypass auf die Kranzader technisch nicht möglich ist. Für diese Patienten ist eine Revaskularisation der Herzkranzgefäße möglich, indem arterielles Blut "von hinten" den Herzkranzgefäßen zugeführt wird, also über den Koronarsinus. Dazu wird, wie dies in Figur 2 schematisch gezeigt ist, ein Konduit 7 durch das Ostium des Koronarsinus in den Koronarsinus 4 eingeführt. Das Konduit 7 erstreckt sich dabei vom Koronarsinus durch den rechten Vorhof und durch die obere Hohlvene 9 in eine große zentrale oder periphere Arterie (in aller Regel die Halsschlagader) 10. Der Übergang vom venösem zum arteriellen Gefäß bereitet dabei keine besonderen Schwierigkeiten und ist übliche chirurgische Praxis, so dass darauf nicht näher eingegangen werden muss. Am distalen Ende 8 muss das Konduit aber im Koronarsinus 4 verankert werden. Diese Verankerung wird man häufig abdichtend vornehmen, da in den Koronarsinus noch kleine Adern 6 führen. In anderen Fällen ist es aber nicht erwünscht, hier eine vollständige Abdichtung zu schaffen, um eine gewisse Rückströmung venösen Blutes zu erhalten.

Verschiedene Arten der Befestigung des distalen Endes 8 sind in den folgenden Figuren gezeigt.

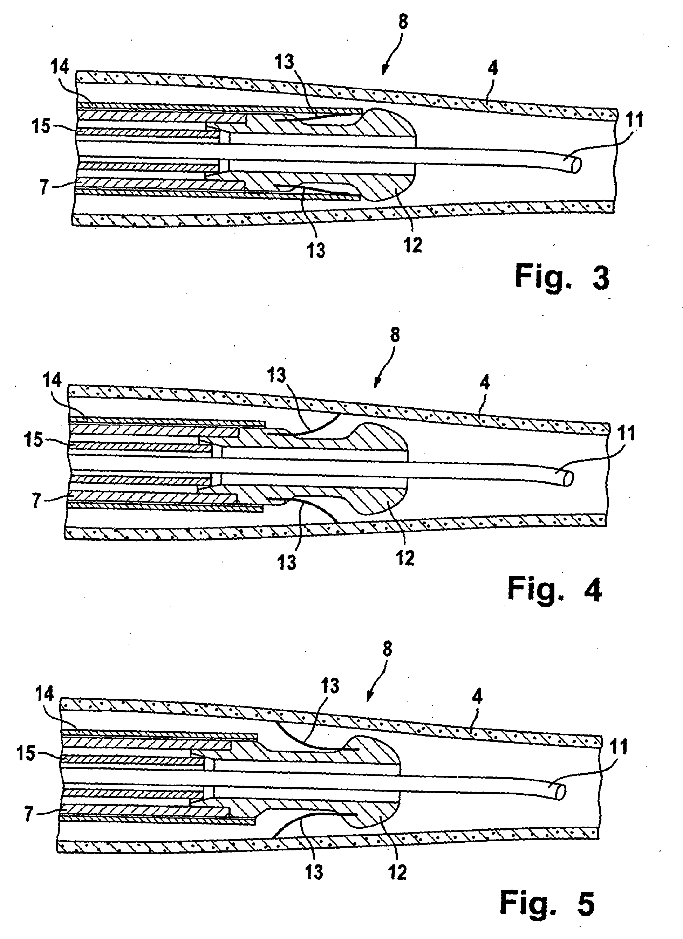

In Fig. 3 ist der distale Endbereich 8 eines Konduits 7 gezeigt, der in eine Ader 4 eingeschoben ist. Zu diesem Zweck war vorher ein Führungsdraht 11 in die Ader 4 eingeschoben worden. Das Konduit 7 ist an seinem distalen Ende 8 mit einem Kopfstück 12 versehen. An diesem sind aussen am Umfang hakenförmige Verankerungselemente 13 vorgesehen, die durch eine aufgeschobene Hülse 14 entgegen ihrer Vorspannung zusammengehalten werden. Am Kopfstück 12 liegt ein röhrenförmiges Element 15 an, das sich bis zum proximalen Ende des Katheters erstreckt. Dieses röhrenförmige Element 15, das im Folgenden auch als Pusher bezeichnet wird, übt eine Gegenkraft auf das Kopfstück 12 aus, wenn die Hülse 14 zurückgezogen wird, um die Verankerungselemente 13 freizugeben. Dadurch wird verhindert, dass beim Zurückziehen der Hülse 14 auch das distale Ende 8 des Konduits trotz der Wirkung der Verankerungselemente 13 mit zurückgezogen wird, wodurch unter anderem die Wand der Ader 4 beschädigt würde und das distale Ende 8 vom gewünschten Ort entfernt würde. Wie dies in Fig. 4 gezeigt ist, spreizen sich die Verankerungselemente 13 aufgrund ihrer elastischen Vorspannung auseinander und verankern sich in der Wand der Ader 4. Ist die Hülse 14 soweit herausgezogen, dass das Konduit freigegeben ist, kann auch der Pusher 15 herausgezogen werden. Durch Wahl entsprechender Materialien oder geeignete Oberflächenbehandlung kann dabei die Reibung gegenüber dem Führungsdraht 11 und dem Konduit 7 ausreichend klein sein, dass auf die Verankerungselement 13 keine schädliche Zugkraft ausgeübt wird.

Bei der Ausführungsform der Fig. 3 und 4 spreizen sich die Verankerungselemente 13 nach vorne, so dass durch erneutes Aufschieben der Hülse 14 die Verankerungselemente 13 wieder zusammengedrückt werden und das distale Ende 8 des Konduits 7 erneut positioniert werden kann. Bei der Ausführungsform der Fig. 5 spreizen sich die Verankerungselemente 13 nach hinten, was einen besseren Halt gegen Zurückziehen ergibt. Der Nachteil ist allerdings, dass die Verankerungselemente 13 durch Aufschieben der Hülse 14 nicht erneut zusammengedrückt werden können.

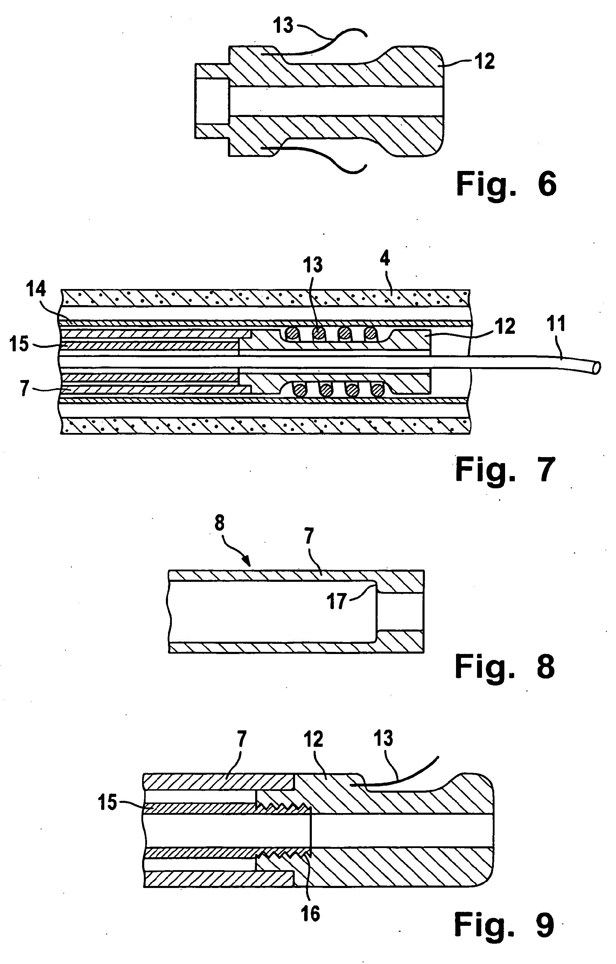

Bei der Ausführungsformen der Fig. 6 sind die im Kopfstück 12 angeordneten Verankerungselemente 13 an ihren Enden abgerundet, so dass die Gefahr der Beschädigung der Wand der Ader 4 geringer ist. Bei der Ausführungsform der Fig. 7 ist ein Verankerungselement 13 in Form einer Spirale im Kopfstück 12 vorgesehen.

Fig. 8 zeigt den distalen Endbereich 8 des Konduits 7, der mit einer Durchmesserverengung 17 versehen ist war, gegen die der Pusher 15 anliegen kann. In Fig. 9 ist schließlich eine Drehverbindungseinrichtung 16 gezeigt wird, mit der der Pusher 15 und das Kopfstück 12 durch Drehen des Pushers 15 so miteinander verbunden werden können, dass durch den Pusher 15 auch eine Zugkraft auf das Kopfstück 12 ausgeübt werden kann. Diese Drehverbindung kann einerseits ein Gewinde sein. Andererseits könnte eine Bajonettverbindung vorgesehen sein. In dieser Figur ist auch gezeigt, dass die Verankerungselemente 13 nur auf einer Seite des Kopfstücks 12 angeordnet sind.

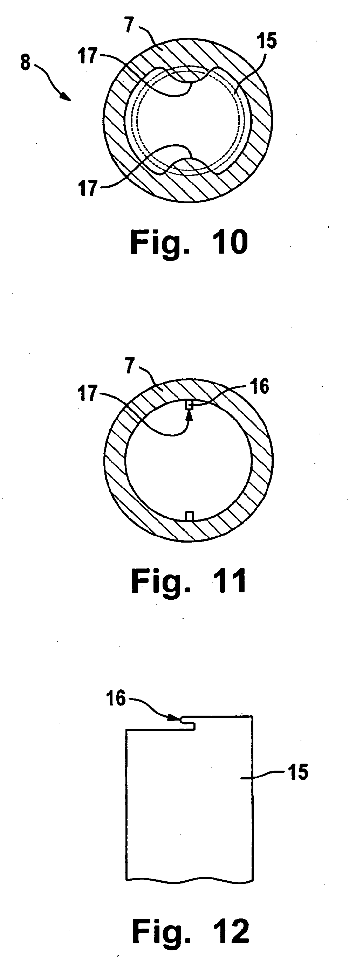

Fig. 10 zeigt im Querschnitt den Endbereich 8 des Konduits 7. Die Durchmesserverengung 17 erstreckt sich dabei nicht um den ganzen Innenumfang des Konduits, sondern besteht im wesentlichen aus zwei Vorsprüngen, gegen die der Pusher 15 anliegt. Die Durchmesserverengung 17 engt so das Lumen weniger ein und behindert den Blutstrom weniger.

In Fig. 11 ist eine ähnliche Darstellung wie in Fig. 9 gezeigt. Die Verengung 17 im besteht hier im wesentlichen aus stiftförmigen Vorsprüngen 16 eines Bajonettverschlusses, dessen komplementäre Ausnehmungen am Ende des röhrenförmigen Elementes 15 in Figur 12 gezeigt sind.

## Patentansprüche

1. In eine Ader (4) des Blutkreislaufs einführbarer Katheter, insbesondere zur Myokardrevaskularisation durch partielle Arterialisierung des Koronarsinus, der ein Konduit (7) mit wenigstens einem expandierbaren Verankerungselement (13) an seinem distalen Ende (8) zum Verankern an der Aderwand und eine schlauchförmige Hülse (Sheet 14) aufweist, die auf das Konduit (7) aufgeschoben ist, nach Einbringung des Konduits (7) in die Ader (4) nach proximal wenigstens teilweise zurückziehbar ist und dabei das wenigstens eine Verankerungselement (13) freigibt, so dass dieses expandieren kann, **dadurch gekennzeichnet, dass** das Konduit (7) an seinem distalen Ende (8) mit einer Verengung (17) seines Lumens versehen ist und der Katheter ein auf einen Führungsdraht (11) aufschiebbares röhrenförmiges Element (Pusher 15) aufweist, das an seinem vorderen Ende gegen die Verengung (17) anliegt.

2. Katheter nach Anspruch 1, **dadurch gekennzeichnet, dass** mehrere Verankerungselemente (13) in Form von federnden Elementen, insbesondere elastischen Haken vorgesehen sind.

3. Katheter nach Anspruch 2, **dadurch gekennzeichnet, dass** die Haken (13) sich vom Konduit (7) nach distal und nach außen erstrecken.

4. Katheter nach Anspruch 2, **dadurch gekennzeichnet, dass** sich die Haken (13) vom Konduit (7) nach proximal und nach außen erstrecken.

5. Katheter nach einem der Ansprüche 2 bis 4, **dadurch gekennzeichnete, dass** die Haken (13) an ihrem vorderen Ende abgerundet sind.

6. Katheter nach einem der Ansprüche 2 bis 5, **dadurch gekennzeichnet, dass** die Haken (13) nur auf einer Seite des Umfangs des Konduits angeordnet sind.

7. Katheter nach Anspruch 1, **dadurch gekennzeichnet, dass** das Verankerungselement (13) eine Spirale ist.

8. Katheter nach einem der Ansprüche 1 bis 7, **dadurch gekennzeichnet, dass** das röhrenförmige Element (Pusher 15) aus Kunststoff oder kunststoffartigem Material ist.

9. Katheter nach einem der Ansprüche 1 bis 8, **dadurch gekennzeichnet, dass** das röhrenförmige Element (Pusher 15) aus Polytetrafluorethylen ist.

10. Katheter nach einem der Ansprüche 1 bis 9, **dadurch gekennzeichnet, dass** das röhrenförmige Element (15) aus flexiblem, dünnwandigen Metall besteht.

11. Katheter nach einem der Ansprüche 1 bis 10, **dadurch gekennzeichnet, dass** die Verengung (17) des Lumens im distalen Endbereich (8) des Konduits (7) ausgebildet ist.

12. Katheter nach einem der Ansprüche 1 bis 10, **dadurch gekennzeichnet, dass** das Konduit (7) an seinem distalen Ende (8) ein mit der Verengung (17) versehenes Kopfstück (12) aufweist.

13. Katheter nach einem der Ansprüche 1 bis 12, **dadurch gekennzeichnet, dass** die Verengung (17) und das distal Ende des röhrenförmigen Elements (Pusher 15) mit komplementären Drehverbindungseinrichtungen (16) versehen sind.

14. Katheter nach Anspruch 13, **dadurch gekennzeichnet, dass** die komplementären Drehverbindungseinrichtungen (16) Gewinde sind.

15. Katheter nach Anspruch 13, **dadurch gekennzeichnet, dass** die komplementären Drehverbindungseinrichtungen (16) Bajonettverbindungen sind.

16. Katheter nach einem der Ansprüche 1 bis 15, **dadurch gekennzeichnet, dass** sich die Verengung (17) und/oder die Drehverbindungseinrichtungen (16) nur über einen Teil des Innenumfangs des Konduits (7) erstrecken.

## Claims

1. Catheter insertable in a blood vessel (4), in particular for myocardial revascularization by partial arterialization of the coronary sinus, which catheter has a conduit (7), with at least one expandable anchoring element (13) at the distal end (8) thereof for anchoring on the vessel wall, and a tubular sleeve (sheet 14) which is pushed onto the conduit (7), can be drawn back at least partially in the proximal direction, after introduction of the conduit (7) into the vessel (4), and in so doing releases the at least one anchoring element (13) such that the latter can expand, **characterized in that** the conduit (7) is provided at its distal end (8) with a constriction (17) of its lumen, and the catheter has a tubular element (pusher 15) which can be pushed onto a guide wire (11) and which bears at its front end against the constriction (17).

2. Catheter according to Claim 1, **characterized in that** several anchoring elements (13) in the form of resilient elements, in particular elastic hooks, are provided.

3. Catheter according to Claim 2, **characterized in that** the hooks (13) extend distally and outwards from the conduit (7).

4. Catheter according to Claim 2, **characterized in that** the hooks (13) extend proximally and outwards from the conduit (7).

5. Catheter according to one of Claims 2 to 4, **characterized in that** the hooks (13) are rounded at their front end.

6. Catheter according to one of Claims 2 to 5, **characterized in that** the hooks (13) are arranged only on one side of the circumference of the conduit.

7. Catheter according to Claim 1, **characterized in that** the anchoring element (13) is a coil.

8. Catheter according to one of Claims 1 to 7, **characterized in that** the tubular element (pusher 15) is made of plastic or plastic-like material.

9. Catheter according to one of Claims 1 to 8, **characterized in that** the tubular element (pusher 15) is made of polytetrafluoroethylene.

10. Catheter according to one of Claims 1 to 9, **characterized in that** the tubular element (15) is made of flexible thin metal.

11. Catheter according to one of Claims 1 to 10, **characterized in that** the constriction (17) of the lumen is formed in the distal end area (8) of the conduit (7).

12. Catheter according to one of Claims 1 to 10, **characterized in that** the conduit (7) has, at its distal end (8), a head piece (12) provided with the constriction (17).

13. Catheter according to one of Claims 1 to 12, **characterized in that** the constriction (17) and the distal end of the tubular element (pusher 15) are provided with complementary rotary connection means (16).

14. Catheter according to Claim 13, **characterized in that** the complementary rotary connection means (16) are threads.

15. Catheter according to Claim 13, **characterized in that** the complementary rotary connection means (16) are bayonet connections.

16. Catheter according to one of Claims 1 to 15, **characterized in that** the constriction (17) and/or the rotary connection means (16) extend over only part of the inner circumference of the conduit (7).

## Revendications

1. Cathéter implantable dans une veine (4) de la circulation sanguine, en particulier pour la vascularisation du myocarde par artérialisation partielle du sinus coronaire, qui comporte un conduit (7) avec au moins un élément d'ancrage (13) expansible au niveau de son extrémité distale (8) pour l'ancrage à la paroi veineuse et une gaine tubulaire (sheet 14), qui est emmanchée sur le conduit (7), qui peut être retirée au moins partiellement vers le côté proximal après l'introduction du conduit (7) dans la veine (4) et, à cette occasion, libère ledit au moins un élément d'ancrage (13), de telle sorte que celui-ci peut se détendre, **caractérisé en ce que** le conduit (7) est muni au niveau de son extrémité distale (8) d'un rétrécissement (17) de son ouverture et le cathéter comporte un élément tubulaire (pusher 15) qui peut être emmanché sur un fil guide (11) et qui est en appui avec son extrémité avant contre le rétrécissement (17).

2. Cathéter selon la revendication 1, **caractérisé en ce qu'**il est prévu plusieurs éléments d'ancrage (13) en forme d'éléments flexibles, en particulier des crochets élastiques.

3. Cathéter selon la revendication 2, **caractérisé en ce que** les crochets (13) s'étendent depuis le conduit (7) vers le côté distal et vers l'extérieur.

4. Cathéter selon la revendication 2, **caractérisé en ce que** les crochets (13) s'étendent depuis le conduit (7) vers le côté proximal et vers l'extérieur.

5. Cathéter selon l'une quelconque des revendications 2 à 4, **caractérisé en ce que** les crochets (13) sont arrondis au niveau de leur extrémité avant.

6. Cathéter selon l'une quelconque des revendications 2 à 5, **caractérisé en ce que** les crochets (13) sont disposés uniquement sur un côté de la périphérie du conduit.

7. Cathéter selon la revendication 1, **caractérisé en ce que** l'élément d'ancrage (13) est une spirale.

8. Cathéter selon l'une quelconque des revendications 1 à 7, **caractérisé en ce que** l'élément tubulaire (pusher 15) est réalisé dans une matière plastique ou un matériau similaire à la matière plastique.

9. Cathéter selon l'une quelconque des revendications 1 à 8, **caractérisé en ce que** l'élément tubulaire (pusher 15) est réalisé en polytetrafluoréthylène.

10. Cathéter selon l'une quelconque des revendications 1 à 9, **caractérisé en ce que** l'élément tubulaire (pusher 15) est réalisé en métal flexible, à paroi mince.

11. Cathéter selon l'une quelconque des revendications 1 à 10, **caractérisé en ce que** le rétrécissement (17) de l'ouverture est réalisé dans la zone de l'extrémité distale (8) du conduit (7).

12. Cathéter selon l'une quelconque des revendications 1 à 10, **caractérisé en ce que** le conduit (7) comporte, au niveau de son extrémité distale (8), une partie formant tête (12) munie du rétrécissement (17).

13. Cathéter selon l'une quelconque des revendications 1 à 12, **caractérisé en ce que** le rétrécissement (17) et l'extrémité distale de l'élément tubulaire (pusher 15) sont munis de dispositifs de liaison rotatifs (16) complémentaires.

14. Cathéter selon la revendication 13, **caractérisé en ce que** les dispositifs de liaison rotatifs (16) complémentaires sont des filetages.

15. Cathéter selon la revendication 13, **caractérisé en ce que** les dispositifs de liaison rotatifs (16) complémentaires sont des assemblages à baïonnette.

16. Cathéter selon l'une quelconque des revendications 1 à 15, **caractérisé en ce que** le rétrécissement (17) et/ou les dispositifs de liaison rotatifs (16) complémentaires s'étendent uniquement sur une partie de la périphérie intérieure du conduit (7).
